# EUROPÄISCHE PATENTSCHRIFT

(11) **EP 0 801 641 B1**
(45) Veröffentlichungstag und Bekanntmachung des Hinweises auf die Patenterteilung: **05.03.2003**
(21) Anmeldenummer: 95942195.9
(22) Anmeldetag: 22.12.1995
(51) Int. Cl.: C07C 253/30, C07C 255/24

(54) **VERFAHREN ZUR HERSTELLUNG VON ALIPHATISCHEN ALPHA,OMEGA-AMINONITRILEN IN DER GASPHASE**
PROCESS FOR PREPARING ALIPHATIC ALPHA,OMEGA-AMINONITRILES IN THE GASEOUS PHASE
PROCEDE DE PRODUCTION D'ALPHA, OMEGA-AMINONITRILES ALIPHATIQUES EN PHASE GAZEUSE

(30) Priorität: 03.01.1995 DE 19500040
(43) Veröffentlichungstag der Anmeldung: 22.10.1997
(73) Patentinhaber: BASF AKTIENGESELLSCHAFT, 67056 Ludwigshafen (DE)
(72) Erfinder: SCHNURR, Werner, D-67273 Herxheim (DE); WULFF-DÖRING, Joachim, D-67227 Frankenthal (DE); FISCHER, Rolf, D-69121 Heidelberg (DE); BÄZNER, Rudolf, D-68259 Mannheim (DE)
(86) Internationale Anmeldenummer: EP9505104
(87) Internationale Veröffentlichungsnummer: WO96020916

(56) Entgegenhaltungen:
- WO-A-94/26699
- JOURNAL OF THE CHEMICAL SOCIETY. FARADAY TRANSACTIONS, Bd. 89, Nr. 21, 7.November 1993 Seiten 3981-3986, XP 000404037 MEDINA F ET AL 'STRUCTURAL AND CATALYTIC PROPERTIES OF SEVERAL POTASSIUM-DOPED NICKEL/ -ALUMINA SOLIDS' in der Anmeldung erwähnt
- JOURNAL OF THE CHEMICAL SOCIETY. FARADAY TRANSACTIONS, Bd. 89, Nr. 18, 21.September 1993 Seiten 3507-3512, XP 000387156 MEDINA F ET AL 'SURFACE CHARACTERIZATION AND HYDROGENATION PROPERTIES OF SEVERAL NICKEL/A-ALUMINA CATALYSTS'
- CHEMICAL ABSTRACTS, vol. 118, no. 6, 8.Februar 1993 Columbus, Ohio, US; abstract no. 41174, MEDINA, F. ET AL 'Characterization and catalytic properties of several potassium-doped iron-nickel catalysts' & APPL. CATAL., A (1992), 92(2), 131-41 CODEN: ACAGE4, 1992
- CHEMICAL ABSTRACTS, vol. 110, no. 7, 13.Februar 1989 Columbus, Ohio, US; abstract no. 56893, MARES, FRANK ET AL 'Preparation and characterization of a novel catalyst for the hydrogenation of dinitriles to amino nitriles' & J. CATAL. (1988), 112(1), 145-56 CODEN: JCTLA5;ISSN: 0021-9517, 1988

## Beschreibung

Die vorliegende Erfindung betrifft ein verbessertes Verfahren zur Herstellung von aliphatischen alpha,omega-Aminonitrilen durch partielle Hydrierung von aliphatischen alpha,omega-Dinitrilen bei erhöhter Temperatur in Gegenwart eines Katalysators in der Gasphase.

In J. Mol. Cat. 61, (1990) S. 197 bis 205 wird die Gasphasenhydrierung von Adiponitril an mit Alkalimetall dotierten Eisenund Nickelkatalysatoren in Abwesenheit von Ammoniak beschrieben. Des weiteren wird in J. Chem. Soc. Faraday Trans. 89 (1993) S. 3981 bis 3986 die partielle Hydrierung von Adipodinitril an Nickel-haltigen Katalysatoren mit 30 Gew.-% Nickel, die mit Kalium dotiert sind, beschrieben. Bei einer Reaktionstemperatur von 170°C und Normaldruck werden Ausbeuten bis zu 85% bei einem Umsatz von 65% erhalten. Nachteilig an diesen Methoden ist allerdings, daß die Katalysatoren pulverförmig sind und daher für eine großtechnische Realisierung in einem Festbett, insbesondere aufgrund der geringen Standzeit der Katalysatoren, nicht in Frage kommen. Ferner wird ein hoher Wasserstoff-Überschuß verwendet und auch die Katalysatorbelastung mit 0.02 kg Dinitril/ l Katalysator und Stunde ist für eine wirtschaftliche Nutzung unbrauchbar.

Aufgabe der vorliegenden war daher die Bereitstellung eines verbesserten Verfahrens zur Herstellung von aliphatischen alpha,omega-Aminonitrilen durch partielle Hydrierung von Adipodinitril, das die zuvor genannten Nachteile nicht aufweist. Insbesondere sollte ein Katalysator gefunden werden, der eine längere Standzeit als die bislang verwendeten Katalysatoren aufweist.

Demgemäß wurde ein Verfahren zur Herstellung von aliphatischen alpha,omega-Aminonitrilen durch partielle Hydrierung von aliphatischen alpha,omega-Dinitrilen bei einer Temperatur im Bereich von 100 bis 250°C, einem Druck im Bereich von 0,01 bis 3 MPa und einem molaren Verhältnis von Wasserstoff zu Dinitril im Bereich von 2:1 bis 300:1 in Gegenwart eines Katalysators in der Gasphase gefunden, indem man die Hydrierung in einem Festbettreaktor mit einem Formkörper-Katalysator auf der Basis mindestens eines Metalls, ausgewählt aus der Gruppe bestehend aus Nickel, Cobalt, Ruthenium und Rhodium, durchführt mit der Maßgabe, daß der Katalysator kein Pulver ist und daß die Katalysatorbelastung 0,03 bis 10 kg Dinitril je kg Katalysator und Stunde beträgt.

Als Ausgangsstoffe im erfindungsgemäßen Verfahren werden aliphatische alpha,omega-Dinitrile der allgemeinen Formel I

NC-(CH₂)ₙ-CN I

in der n eine ganze Zahl von 1 bis 10, insbesondere 2, 3, 4, 5 und 6, bedeutet, eingesetzt. Besonders bevorzugte Verbindungen I sind Bernsteinsäuredinitril, Glutarsäuredinitril, Adipinsäuredinitril ("Adiponitril"), Pimelinsäuredinitril und Korksäuredinitril ("Suberonitril"), ganz besonders bevorzugt Adiponitril.

Nach dem erfindungsgemäßen Verfahren werden die vorstehend beschriebenen Dinitrile I in Gegenwart eines Katalysators partiell zu alpha,omega-Aminonitrilen der allgemeinen Formel II

NC-(CH₂)ₙ-CH₂-H₂ II

hydriert, wobei n die vorstehend genannte Bedeutung hat. Besonders bevorzugte Aminonitrile II sind solche, in denen n einen Wert von 2, 3, 4, 5 oder 6 hat, insbesondere 4, d.h. 4-Aminobutansäurenitril, 5-Aminopentansäurenitril, 6-Aminohexansäurenitril ("6-Aminocapronitril"), 7-Aminoheptansäurenitril und 8-Aminooctansäurenitril, ganz besonders bevorzugt 6-Aminocapronitril.

Die Umsetzung nimmt man erfindungsgsgemäß bei Temperaturen im Bereich von 100 bis 250, vorzugsweise von 150 bis 220, besonders vorzugsweise von 160 bis 200°C vor; den Druck wählt man erfindungsgemäß im Bereich von 0,01 bis 3, vorzugsweise von 0,07 bis 1, besonders bevorzugt von 0,09 bis 0,5 MPa.

Die Wasserstoffkonzentration im Eingangsgas hängt im wesentlichen von der Dinitrilkonzentration ab. Erfindungsgemäß wählt man das Molverhältnis von Wasserstoff zu Dinitril im Bereich von 2:1 bis 300:1, bevorzugt von 10:1 bis 200:1

In einer bevorzugten Ausführungsform setzt man zusätzlich Ammoniak oder ein Amin, besonders bevorzugt Ammoniak, ein. Insbesondere bei Verwendung von Ammoniak wird nach bisherigen Beobachtungen eine Verlängerung der Katalysatorstandzeit erzielt. Üblicherweise beträgt die Menge an Ammoniak oder Amin 5 bis 50, bevorzugt 10 bis 30 Gew.-%, bezogen auf eingesetztes Dinitril.

Als Katalysatoren setzt man erfindungsgemäß solche auf der Basis mindestens eines Metalls, ausgewählt aus der Gruppe bestehend aus Nickel, Cobalt, Ruthenium und Rhodium, ein, bevorzugt solche auf der Basis von Nickel.

Man kann die Katalysatoren als Voll- oder Trägerkatalysatoren einsetzen, wobei als Träger beispielsweise Aluminiumoxid, Siliciumdioxid, Alumosilikate, Titandioxid, Zirkondioxid, Magnesiumoxid, bevorzugt Aluminiumoxid, Siliciumdioxid, Alumosilikate, besonders bevorzugt Aluminiumoxid, dienen können.

Die Katalysatoren können in einer bevorzugten Ausführungsform mit einem Modifizierungsmittel auf der Basis eines Metalls, ausgewählt aus der Gruppe aus Lithium, Natrium, Kalium, Rubidium, Cäsium, Magnesium, Calcium, Strontium, Barium, Scandium, Yttrium, Lanthaniden, Silber, Cadmium, Aluminium, Zinn und Zink, vorzugsweise mit deren Oxiden, modifiziert ein Besonders bevorzugt sind Alkalimetalloxide wie Lithium-, Natrium-, Kalium-, Rubidiumund Cäsiumoxid und Zinkoxid, ganz besonders bevorzugt Cäsiumoxid.

Die Menge an Katalysator wählt man erfindungsgemäß so, daß die Katalysatorbelastung im Bereich von 0,03 bis 10, vorzugsweise von 0,05 bis 3 kg Dinitril je kg Katalysator und Stunde liegt.

Bei Verwendung von Trägerkatalysatoren wählt man üblicherweise den Metallgehalt des Katalysators im Bereich von 0,1 bis 80, bevorzugt von 0,2 bis 70, besonders bevorzugt von 0,5 bis 50 Gew.-%, bezogen auf das Trägermaterial.

Erfindungsgemäß setzt man die Katalysatoren nicht als Pulver ein, sondern als Formkörper wie Stränge, Tabletten und Kugeln, je nach vorgesehenem Verwendungszweck.

Die Umsetzung führt man, bevorzugt kontinuierlich, erfindungsgemäß als Festbettreaktion mit fest angeordnetem Katalysator durch, beispielsweise in Sumpf- oder Rieselfahrweise. Durch Veränderung der Verweilzeit kann man den Umsatz und damit die Selektivität gezielt einstellen.

Die Herstellung der Katalysatoren ist an sich bekannt (s. Appl. Het. Cat., 1987, S. 106-122; Catalysis, Vol. 4 (1981) S. 1-30; DE-A 2 260 978). Üblicherweise erfolgt die Herstellung derart, daß man wasserlösliche Salze wie Nitrate, Sulfate, Chloride, Formiate oder Acetate der entsprechenden Metalle in Gegenwart oder Abwesenheit eines Trägermaterials ausfällt, gewünschtenfalls den so erhaltenen Katalysatorvorläufer zu Strängen oder Tabletten verarbeitet, trocknet und anschließend calciniert. Trägerkatalysatoren sind im allgemeinen auch erhältlich, indem man den Träger mit einem wäßrigen Salz des entsprechenden Metalls oder der entsprechenden Metalle tränkt, gewünschtenfalls in Gegenwart der zuvor genannten Modifizierungsmittel, insbesondere Cäsiumverbindungen, oder indem man die entsprechenden Metallsalzlösungen auf das Trägermaterial aufsprüht.

Die Fällung erfolgt im allgemeinen wahlweise durch Zugabe von Fällungsreagenzien, durch Änderung des pH-Wertes oder durch Änderung der Temperatur.

Üblicherweise trocknet man die so erhaltene Katalysatorvormasse im allgemeinen bei Temperaturen im Bereich von 80 bis 150, vorzugsweise von 80 bis 120°C vor.

Das Calcinieren nimmt man in der Regel bei Temperaturen im Bereich von 150 bis 700, vorzugsweise von 200 bis 650°C in einer Gasmischung aus Wasserstoff und Stickstoff vor.

Nach dem erfindungsgemäßen Verfahren zeigen die eingesetzten Katalysatoren, insbesondere Trägerkatalysatoren, über einen längeren Zeitraum als bislang üblich eine hohe Aktivität. Man erhält alpha,omega-Aminonitrile in hoher Ausbeute und in guten Selektivitäten. Die alpha,omega-Aminonitrile sind wichtige Ausgangsverbindungen zur Herstellung von cyclischen Lactamen, insbesondere 6-Aminocapronitril für Caprolactam.

### Beispiele

Pro Stunde wurden 3 bis 20 g Adipinsäuredinitrilin einen Verdampfer (300°C) geleitet und von dort mit 100 bis 200 l/h Wasserstoff über 100 ml Katalysator in Rielfahrweise geleitet. Der gasförmige Reaktionsaustrag wurde Kühlfallen kondensiert und gaschromatographisch analysiert. Weitere Reaktionsparameter sowie die Ergebnisse finden sich in untenstehender Tabelle.

### Beispiel 1

225 g Aluminiumoxid-Stränge (Durchmesser 4mm; BET-Oberfläche 1 m²/g) wurden mit 67 ml einer wäßrigen Nickelnitrat-Lösung (Gehalt an Nickel: 16,72 g, berechnet als Nickelmetall) versetzt und zwei Stunden lang bei Raumtemperatur unter mehrmaligem, guten Rühren stehengelassen. Anschließend wurde der so erhaltene Katalysatorvorläufer 16 h bei 120°C getrocknet und 4 h bei 350°C calciniert. Danach wurde der gesamte Vorgang wiederholt.

Nach dem Abkühlen wurden die Stränge in eine Reduktionsapparatur eingebaut und 2 h mit 20 l Stickstoff/h gespült. Danach wurde mit 2°C/min und 20 l Wasserstoff/h auf 300°C aufgeheizt und 20 h bei dieser Temperatur gehalten. Nach Abkühlen im Stickstoffstrom wurde der Katalysator mit einem Luft/Stickstoff-Gemisch passiviert, wobei die Temperaturerhöhung maximal 20°C betrug.

Die erhaltenen Katalysatorstränge enthielten 13 Gew.-% Nickel (gerechnet als Metall, bezogen auf Aluminiumoxid).

### Beispiel 2

Beispiel 1 wurde wiederholt mit dem Unterschied, daß vor der Tränkung mit Nickelnitrat eine Tränkung mit Lithiumnitrat (32 ml einer 3,4 gew.-%igen Lösung) durchgeführt wurde.

Die erhaltenen Katalysatorstränge enthielten 13 Gew.-% Nickel (gerechnet als Metall, bezogen auf Aluminiumoxid) und 0,1 Gew.-% Lithium (gerechnet als Metall, bezogen auf Aluminiumoxid).

### Beispiel 3

Beispiel 1 wurde wiederholt mit dem Unterschied, daß vor der Tränkung mit Nickelnitrat eine Tränkung mit Natriumnitrat (32 ml einer 1,4 gew.-%igen Lösung) durchgeführt wurde.

Die erhaltenen Katalysatorstränge enthielten 13 Gew.-% Nickel (gerechnet als Metall, bezogen auf Aluminiumoxid) und 0,1 Gew.-% Natrium (gerechnet als Metall, bezogen auf Aluminiumoxid).

### Beispiel 4

Beispiel 1 wurde wiederholt mit dem Unterschied, daß vor der Tränkung mit Nickelnitrat eine Tränkung mit Kaliumnitrat (32 ml einer 1,0 gew.-%igen Lösung) durchgeführt wurde.

Die erhaltenen Katalysatorstränge enthielten 13 Gew.-% Nickel (gerechnet als Metall, bezogen auf Aluminiumoxid) und 0,1 Gew.-% Kalium (gerechnet als Metall, bezogen auf Aluminiumoxid).

### Beispiel 5

Beispiel 1 wurde wiederholt mit dem Unterschied, daß vor der Tränkung mit Nickelnitrat eine Tränkung mit Cäsiumnitrat (32 ml einer 0,5 gew.-%igen Lösung) durchgeführt wurde.

Die erhaltenen Katalysatorstränge enthielten 13 Gew.-% Nickel (gerechnet als Metall, bezogen auf Aluminiumoxid) und 0,1 Gew.-% Cäsium (gerechnet als Metall, bezogen auf Aluminiumoxid).

### Beispiel 6

Beispiel 5 wurde mit einem Aluminiumoxid, das eine BET-Oberfläche von 5,5 m²/g aufwies (SCS 9 (Pechiney)), wiederholt.

Die erhaltenen Katalysatorstränge enthielten 13 Gew.-% Nickel (gerechnet als Metall, bezogen auf Aluminiumoxid) und 0,1 Gew.-% Cäsium (gerechnet als Metall, bezogen auf Aluminiumoxid).

### Beispiel 7

Beispiel 5 wurde mit einem Aluminiumoxid, das eine BET-Oberfläche von 7,7 m²/g aufwies (SPH 512 B (Rhone-Poulenc)), wiederholt.

Die erhaltenen Katalysatorstränge enthielten 13 Gew.-% Nickel (gerechnet als Metall, bezogen auf Aluminiumoxid) und 0,1 Gew.-% Cäsium (gerechnet als Metall, bezogen auf Aluminiumoxid).

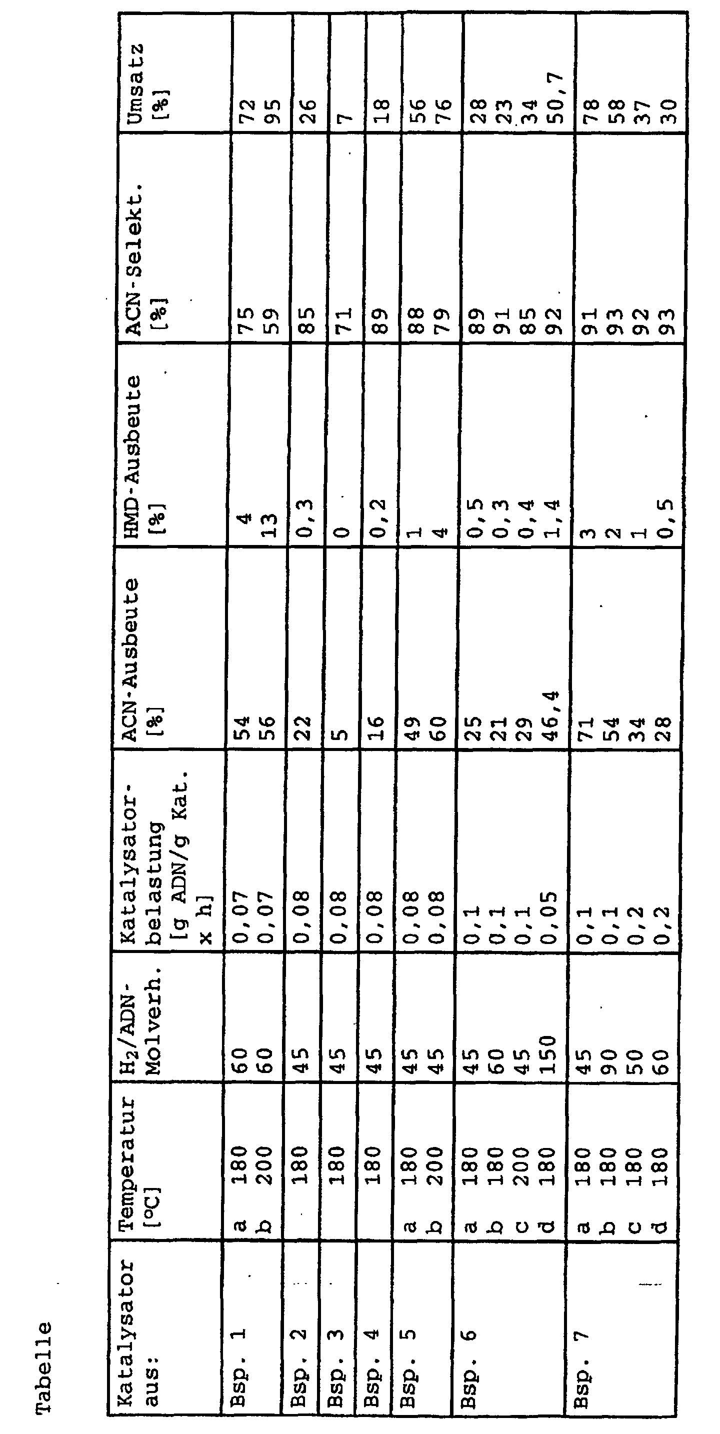

## Patentansprüche

1. Verfahren zur Herstellung von aliphatischen alpha, omega-Aminonitrilen durch partielle Hydrierung von aliphatischen alpha, omega-Dinitrilen bei einer Temperatur im Bereich von 100 bis 250°C, einem Druck im Bereich von 0,01 bis 3 MPa und einem molaren Verhältnis von Wasserstoff zu Dinitril im Bereich von 2:1 bis 300:1 in Gegenwart eines Katalysators in der Gasphase, **dadurch gekennzeichnet, daß** man die Hydrierung in einem Festbettreaktor mit einem Formkörper-Katalysator auf der Basis mindestens eines Metalls, ausgewählt aus der Gruppe bestehend aus Nickel, Cobalt, Ruthenium und Rhodium durchführt mit der Maßgabe, daß der Katalysator kein Pulver ist und daß die Katalysatorbelastung 0,03 bis 10 kg Dinitril je kg Katalysator und Stunde beträgt.

2. Verfahren nach Anspruch 1, **dadurch gekennzeichnet, daß** der Katalysator mit mindestens einem Modifizierungsmittel auf der Basis eines Metalls, ausgewählt aus der Gruppe bestehend aus Lithium, Natrium, Kalium, Rubidium, Cäsium, Magnesium, Calcium, Strontium, Barium, Scandium, Yttrium, Lanthaniden, Silber, Cadmium, Aluminium, Zinn und Zink modifiziert ist

3. Verfahren nach Anspruch 1 oder 2, **dadurch gekennzeichnet, daß** man als aliphatisches alpha, omega-Dinitril Adipodinitril einsetzt unter Erhalt von 6-Aminocapronitril.

## Claims

1. A process for the preparation of aliphatic alpha,omega-aminonitriles by partial hydrogenation of aliphatic alpha,omega-dinitriles at from 100 to 250°C, from 0.01 to 3 MPa and a molar ratio of hydrogen to dinitrile in the range from 2:1 to 300:1 in the presence of a catalyst in the gas phase, which comprises carrying out the hydrogenation in a fixed-bed reactor with a molded catalyst based on at least one metal selected from the group consisting of nickel, cobalt, ruthenium and rhodium, with the proviso that the catalyst is not a powder and that the catalyst space velocity is from 0.03 to 10 kg of dinitrile per kg of catalyst per hour.

2. A process as claimed in claim 1, wherein the catalyst is modified with at least one modifier based on a metal selected from the group consisting of lithium, sodium, potassium, rubidium, caesium, magnesium, calcium, strontium, barium, scandium, yttrium, lanthanides, silver, cadmium, aluminum, tin and zinc.

3. A process as claimed in claim 1 or 2, wherein adiponitrile is used as the aliphatic alpha,omega-dinitrile and 6-aminocapronitrile is obtained.

## Revendications

1. Procédé de préparation d'α,ω-aminonitriles aliphatiques par hydrogénation partielle d'α,ω-dinitriles à une température de l'ordre de 100 à 250°C, une pression de l'ordre de 0,01 à 3 MPa et une proportion molaire de l'hydrogène au dinitrile de l'ordre de 2 : 1 à 300 : 1 en présence d'un catalyseur, dans la phase gazeuse, **caractérisé en ce que** l'on entreprend l'hydrogénation dans un réacteur à lit fixe avec un catalyseur de corps façonnés à base d'au moins un métal choisi dans le groupe formé par le nickel, le cobalt, le ruthénium et le rhodium, avec la condition que le catalyseur ne soit pas une poudre et que la charge du catalyseur soit de 0,03 à 10 kg de dinitrile par kg de catalyseur et par heure.

2. Procédé selon la revendication 1, **caractérisé en ce que** le catalyseur est modifié par au moins un agent de modification à base d'un métal choisi dans le groupe formé par le lithium, le sodium, le potassium, le rubidium, le césium, le magnésium, le calcium, le strontium, le baryum, le scandium, l'yttrium, les lanthanides, l'argent, le cadmium, l'aluminium, l'étain et le zinc.

3. Procédé selon la revendication 1 ou 2, **caractérisé en ce que** l'on met en oeuvre en tant que dinitrile α,ω-aliphatique de l'adiponitrile avec obtention de 6-aminocapronitrile.
